# EUROPÄISCHE PATENTSCHRIFT

(11) **EP 1 281 365 B1**
(45) Veröffentlichungstag und Bekanntmachung des Hinweises auf die Patenterteilung: **29.03.2006**
(21) Anmeldenummer: 01118708.5
(22) Anmeldetag: 03.08.2001
(51) Int. Cl.: A61B 17/86, F16B 39/06

(54) **Befestigungsvorrichtung**
Fastening device
Dispositif de fixation

(43) Veröffentlichungstag der Anmeldung: 05.02.2003
(73) Patentinhaber: BrainLAB AG, 85551 Kirchheim/Heimstetten (DE)
(72) Erfinder: Vilsmeier, Stefan, 6330 Kufstein (AT); Birkenbach, Rainer, 85586 Poing (DE); Merlin, Eric, 85622 Feldkirchen (DE)
(74) Vertreter: Schwabe - Sandmair - Marx

(56) Entgegenhaltungen:
- WO-A-00/66045
- US-A- 1 495 687
- US-A- 1 829 293
- US-A- 2 367 399
- US-A- 4 754 749
- US-A- 6 048 151
- US-B1- 6 203 543

## Beschreibung

Die vorliegende Erfindung bezieht sich auf eine Befestigungsvorrichtung und ein Befestigungssystem, insbesondere zum Anbringen eines Positionierungselements, wie zum Beispiel eines Markers, in einem festen definierten Lageverhältnis an einem Objekt wie zum Beispiel einem Knochen.

Bei chirurgischen Eingriffen, zum Beispiel einer Knie- oder Hüftoperation , insbesondere beim Anbringen eines Implantats ist es bei neueren chirurgischen Verfahren erforderlich die genaue Lage einer bestimmten Körperstruktur, zum Beispiel die Lage der Oberschenkel- und Unterschenkelknochen im Raum möglichst exakt zu erfassen, um unter Verwendung geeigneter Navigationsinstrumente einen möglichst präzisen Eingriff durchführen zu können. Dabei kann zum Beispiel ein künstliches Kniegelenk unter Verwendung von präoperativ oder intraoperativ durchgeführten Aufnahmen und geeigneten Berechnungen präzise an dem Oberschenkel- und Unterschenkelknochen positioniert werden, so dass ein optimaler Sitz gewährleistet werden kann. Zur Bestimmung der Position einer Knochenstruktur wird im allgemeinen eine Schraube in den Knochen eingeschraubt und an dieser Schraube wird ein Marker befestigt, wobei aus der von einer Kamera erfassten Position des Markers auf die Position des Knochens rückgeschlossen wird. Treten kleinere Verschiebungen zwischen dem Knochen und dem daran mittels der Schraube befestigten Marker auf, z.B. durch leichtes Verdrehen der Schraube, so wird aus der erfassten Position des Markers auf eine nicht zutreffende Position des Knochens rückgeschlossen, was erhebliche Auswirkungen auf den Operationserfolg haben kann. So können zum Beispiel Abweichungen im Bereich von ein bis zwei Grad zu deutlich merkbaren Fehlpositionierungen eines implantierten Kniegelenkes führen, was sich durch Probleme bei der Funktionstüchtigkeit des Kniegelenks und eine erheblich verkürzte Lebensdauer bemerkbar machen kann, da aufgrund der leichten Fehlpositionierung das Kniegelenk nicht mehr optimal belastet wird.

Insbesondere bei üblicherweise verwendeten Schrauben stellt sich das Problem, dass eine in einen Knochen eingebrachte Schraube versehentlich leicht gedreht wird, so dass ein chirurgischer Eingriff unpräzise durchgeführt wird.

Die US-Patentschrift 6,203,543 offenbart eine Vorrichtung zum lösbaren Befestigen eines Objekts an einem Knochen. Die Vorrichtung umfasst eine Schraube mit einem Schaft, der ein erstes Gewinde zum Einschrauben des Schafts in einen Knochen und ein zweites Gewinde aufweist, auf das eine Mutter aufgeschraubt werden kann. Entlang des Schafts der eingeschraubten Schraube kann eine Hülse geschoben werden, die an ihrer Stirnseite gezackt ist. Durch Aufschrauben einer Mutter auf das zweite Gewinde wird die zwischen Mutter und Knochen befindliche Hülse gegen den Knochen gedrückt. Dabei verankert sich die gezackte Stirnseite der Hülse im Knochen, wodurch die Hülse verdrehfest mit dem Knochen verbunden wird.

Das gleiche Dokument offenbart alternativ eine ähnliche Vorrichtung, die eine Hülse umfasst, bei der anstatt eines gezackten Endes ein von der Mittelachse der Vorrichtung beabstandeter Stift in dem Knochen verankert wird.

Die US-Patentschrift 4,754,749 offenbart eine Knochenschraube mit der zwei Knochen zueinander fixiert werden können. Die Schraube umfasst einen Kopf und einen Schaft. Der Schaft weist an seinem dem Kopf gegenüber liegenden Ende ein Gewinde auf, mit dem die Schraube in einen ersten Knochen geschraubt werden kann. Zwischen dem Gewinde und dem Kopf befindet sich ein gewindefreier Abschnitt, der durch einen zweiten Knochen geführt wird. Die Unterseite des Kopfes drückt beim Einschrauben der Schraube den zweiten Knochen gegen den ersten Knochen. Im Kopf der Schraube sind zwei schräg zur Längsachse der Schraube verlaufende Bohrungen ausgebildet, die an die Unterseite des Kopfes münden. Durch die schrägen Bohrungen kann ein Stift gesteckt und in den zweiten Knochen eingeführt werden, um eine Verdrehung der Schraube zu verhindern.

Es ist eine Aufgabe der vorliegenden Erfindung eine Befestigungsvorrichtung und ein Befestigungssystem vorzuschlagen, welche das präzise und sichere Positionieren mindestens eines Markers an einem Objekt wie z.B. einem Knochen ermöglichen.

Diese Aufgabe wird durch eine Befestigungsvorrichtung und ein Befestigungssystem mit den Merkmalen der unabhängigen Ansprüche gelöst. Vorteilhafte Ausführungsformen ergeben sich aus den Unteransprüchen.

Obwohl das technische Gebiet der Erfindung beispielhaft an Hand von chirurgischen Eingriffen im Bereich des Knies beschrieben wurde, kann die Erfindung in einem weiten Bereich der Chirurgie oder allgemein der Technik eingesetzt werden, zum Beispiel bei Eingriffen im Bereich der Hüfte, der Wirbelsäule oder des Kopfes. Allgemein kann die Erfindung überall dort eingesetzt werden, wo es erforderlich ist ein bestimmtes Element, wie zum Beispiel einen Marker, möglichst ortsfest und verdreh- bzw. verschiebungssicher an einem Objekt, wie zum Beispiel einem Knochen oder einer Knochenstruktur anzubringen.

Die erfindungsgemäße Befestigungsvorrichtung weist mindestens eine Führung für ein Sicherungselement auf. Durch diese Führung kann vor, nach oder gleichzeitig mit dem Einbringen der Befestigungsvorrichtung in zum Beispiel einen Knochen ein Sicherungselement in den Knochen eingebracht werden, um die eingebrachte Befestigungsvorrichtung durch weiteres Einbringen des Sicherungselements gegen ein leichtes Verdrehen zu sichern.

Vorteilhaft ist die Befestigungsvorrichtung länglich und in etwa zylinderförmig ausgebildet und weist bevorzugt einen Schaft auf, wobei ein Teil des Schaftes bevorzugt dazu geeignet ist in einen Knochen eingebracht zu werden. An einem Ende des Schaftes, welches nicht in einen Knochen eingebracht werden soll, ist bevorzugt ein Kopf vorgesehen, welcher geeignet ausgebildet sein kann um zum Beispiel ein Werkzeug zum Einbringen der Befestigungsvorrich-tung anzusetzen und/oder mindestens ein Positionierungselement möglichst ortsfest daran zu befestigen.

Vorteilhaft weist der Schaft einen Eingriffbereich an einem äußeren Ende und einen Zwischenbereich auf, welcher zwischen dem Eingriffbereich und dem Kopf der Befestigungsvorrichtung angeordnet ist. Der Eingriffbereich ist so ausgebildet, dass ein Einbringen der Befestigungsvorrichtung in ein Objekt, wie zum Beispiel einen Knochen ermöglicht wird, wobei nach dem Einbringen auch eine möglichst stabile Befestigung durch den Eingriffbereich sicher gestellt werden soll.

Vorteilhaft ist der Durchmesser des Zwischenbereichs größer als der Durchmesser des Eingriffbereichs, wobei jedoch auch beide Bereiche mit in etwa gleichem Durchmesser ausgebildet werden können oder auch der Eingrifibereich einen kleineren Durchmesser als der Zwischenbereich aufweisen kann. Dabei ist der Übergang zwischen Eingriffbereich und Zwischenbereich vorteilhaft konisch ausgebildet, um einen scharfen Übergang zwischen Eingriffbereich und Zwischenbereich zu vermeiden. Wenn der Eingriffbereich zum Beispiel einen kleineren Durchmesser als der Zwischenbereich hat, kann die Befestigungsvorrichtung zum Beispiel so weit in ein Objekt eingebracht werden, bis aufgrund des größeren Durchmessers des Zwischenbereiches ein weiteres Einbringen der Befestigungsvorrichtung verhindert oder erschwert wird.

Die erfindungsgemäße Führung ist als Bohrung und/oder als Ausnehmung ausgebildet. Bevorzugt kann die Führung auch als eine Kombination aus einer Bohrung bzw. einem Durchgangsloch eines Kopf- oder Schaftbereiches bestehen, wobei andere Schaftbereiche eine Ausnehmung oder eine weitere Bohrung oder auch keine Führung aufweisen können. Dabei ist die Führung vorteilhaft so ausgebildet, dass sie in etwa in Längsrichtung der Befestigungsvorrichtung verläuft, bevorzugt parallel dazu und vorteilhaft mit einem kleinen seitlichen Versatz von zum Beispiel 0,1 bis 10 mm zur Mittelachse des Eingriffbereiches. Allgemein ist es vorteilhaft, wenn die Befestigungsvorrichtung mindestens eine Bohrung aufweist, durch welche ein Sicherungselement durchgeführt und gehalten werden kann, wobei wenn zum Beispiel die Bohrung im Zwischenbereich des Schaftes angeordnet ist und der Abstand zwischen Mittelachse der Bohrung und Mittelachse des Eingriffbereiches kleiner ist als der Durchmesser des Eingriffbereiches, vorteilhaft eine halbrunde oder runde Ausnehmung im Eingriffbereich vorgesehen ist, um das Sicherungselement entlang des Eingriffbereichs zu führen. Erfindungsgemäß kann die Führung für das Sicherungselement sowohl so ausgebildet sein, dass sich der Außenumfang der Führung bzw. eines dann eingebrachten Sicherungselements mit dem Außenumfang des Eingriffbereichs überschneidet, so dass das Sicherungselement bevorzugt entlang des Eingriffbereichs geführt wird und in der Nähe bzw. anliegend an den Eingriffbereich eingebracht wird. Alternativ kann die Führung auch so ausgebildet sein, dass ein einzubringendes Sicherungselement und der Schaft oder Eingriffbereich der Befestigungsvorrichtung nicht unmittelbar aneinander anliegen, sondern einen Abstand zum Beispiel im Bereich von 0,1 bis 10 mm von einander aufweisen, wobei das Sicherungselement z.B. durch eine Bohrung des Kopfes oder eines Schaftbereiches geführt ist..

Vorteilhaft können mehr als eine Führung, also zum Beispiel 2, 3, 4 bis 10 oder mehr Führungen an der Befestigungsvorrichtung vorgesehen sein, um mehrere Sicherungselemente einbringen zu können. Dabei können die Führungen sowohl parallel zueinander und zu einer Mittelachse des Eingriffbereichs ausgebildet sein. Es ist auch möglich die Führungen so anzuordnen, dass Sicherungselemente unter einem bestimmten Winkel zueinander und ggf. auch zu einer Mittelachse des Eingriffbereichs in die Befestigungsvorrichtung eingebracht werden können, um so zum Beispiel eine stabilere Befestigung oder Sicherung der Befestigungsvorrichtung zu erzielen.

Gemäß einer bevorzugten Ausführungsform ist die erfindungsgemäße Befestigungsvorrichtung als Schraube ausgebildet und weist im Eingriffbereich ein Gewinde auf, wobei im Eingriffbereich durch das Gewinde hindurchgehend ein Führungsloch vorgesehen sein kann. Es kann alternativ oder ergänzend zu einem Führungsloch für ein Sicherungselement eine seitliche Ausnehmung im Eingriffbereich vorgesehen sein, so dass der Schraubengang an einer oder mehreren Stellen unterbrochen wird, um ein oder mehr Sicherungselemente entlang des Eingriffbereichs einbringen zu können.

Der Eingriffbereich kann auch als Nagel ausgebildet sein, wobei ebenfalls mindestens eine Führung im Eingriffbereich vorgesehen sein kann. Dabei kann die Außenfläche des Eingriffbereiches im wesentlichen eben sein, wobei zum Beispiel längs der Einbringrichtung eine oder mehrere Kanten vorgesehen sein können, um das Einbringen der Befestigungsvorrichtung zu erleichtern und ein nachträgliches ungewolltes Verdrehen zu erschweren bzw. zu verhindern.

Vorteilhaft ist am äußeren Ende des Einbringbereiches ein spitz zulaufender Bereich ausgebildet, zum Beispiel in Form eines kleines Kegels oder einer Pyramide mit einer oder mehreren seitlichen Kanten, um das Ansetzen und Einbringen der Befestigungsvorrichtung zu erleichtem.

Bevorzugt ist im Bereich des oder am Kopf der Befestigungsvorrichtung ein Verbindungselement, wie zum Beispiel ein Vorsprung oder eine Vertiefung, zum Beispiel eine Einstecköffnung vorgesehen, um zum Beispiel ein Werkzeug zum Einbringen der Befestigungsvorrichtung ansetzen zu können und/oder ein Positionierungselement oder andere gewünschte Objekte möglichst verdrehsicher mit der Befestigungsvorrichtung verbinden zu können. Dazu ist ein Vorsprung und/oder eine Einstecköffnung vorzugsweise nicht rotationssymmetrisch ausgebildet und kann eine oder mehrere Kanten aufweisen, so dass zum Beispiel ein Inbusschlüssel in den Kopf der Befestigungsvorrichtung eingesetzt werden kann, um diese in einen Knochen einzuschrauben und anschließend verdrehsicher zur Befestigungsvorrichtung ein Positionierungselement, wie zum Beispiel ein Referenzstern, darauf befestigt werden kann, nachdem ein Sicherungselement eingebracht wurde. Vorteilhaft können am Kopf auch mehrere Vertiefungen vorgesehen sein, um ein anzubringendes Positionierungselement in einer Vielzahl von Winkelpositionen verdrehgesichert an der Befestigungsvorrichtung an bringen zu können.

Gemäß einem weiteren Aspekt der Erfindung wird ein Befestigungssystem vorgeschlagen, welches aus der oben beschriebenen Befestigungsvorrichtung besteht, wobei mindestens ein Sicherungselement in die mindestens eine Führung der Befestigungsvorrichtung eingebracht ist. Das Sicherungselement kann zum Beispiel als Schraube ausgebildet sein und weist vorteilhaft eine zylinderförmige längliche Form auf, wobei der Außendurchmesser des Sicherungselements bevorzugt so ausgebildet ist, dass das Sicherungselement mit nur geringem oder ohne Spiel durch die Führung der Befestigungsvorrichtung geführt werden kann. Das Sicherungselement kann ebenso wie die oben beschriebene Befestigungsvorrichtung einen Kopf und einen Schaft aufweisen, welcher zum Beispiel wiederum in Eingriffbereich und Zwischenbereich unterteilt ist. Bezüglich möglicher Ausgestaltungen des Schaftes und/oder Kopfes wird auf die oben für die Befestigungsvorrichtung beschriebenen Merkmale verwiesen, welche ebenso bei dem Sicherungselement ausgebildet sein können.

Bevorzugt ist der Durchmesser des Sicherungselements kleiner als der Durchmesser der Befestigungsvorrichtung, insbesondere kleiner als der Durchmesser des Eingriffsbereichs davon.

Gemäß einem weiteren Aspekt der Erfindung wird ein Positionierungssystem vorgeschlagen, welches aus dem oben beschriebenen Befestigungssystem besteht und weiterhin ein daran befestigtes Positionierungselement, wie zum Beispiel mindestens einen Marker oder einen Referenzstern mit drei Markern bzw. Haltevorrichtungen für die entsprechenden Marker aufweist.

Vorteilhaft ist das Positionierungssystem so ausgebildet, dass das an dem Befestigungssystem angebrachte Positionierungselement verstellt werden kann, zum Beispiel um mindestens eine Achse gedreht werden kann, so dass eine möglichst optimale Ausrichtung des Positionierungselements eingestellt werden kann. Dabei kann das Positionierungselement bevorzugt in der eingestellten Position arretiert werden, so dass ein unbeabsichtigtes Verkippen oder Verschwenken des Positionierungselements relativ zum Befestigungssystem im wesentlichen verhindert wird.

Die Erfindung wird nachfolgend anhand eines bevorzugten Ausführungsbeispiels beschrieben. Es zeigen:
- Figuren 1a bis 1i: eine erfindungsgemäße Ausführungsform einer Befestigungsvorrichtung mit einer Führung;
- Figuren 2a bis 2d: eine Ausführungsform eines Sicherungselements, welches in die Befestigungsvorrichtung von Figur 1 eingebracht werden kann;
- Figuren 3a bis 3c: ein erfindungsgemäßes Positionierungssystem; und
- Figur 4: eine alternative Ausführungsform einer Befestigungsvorrichtung.

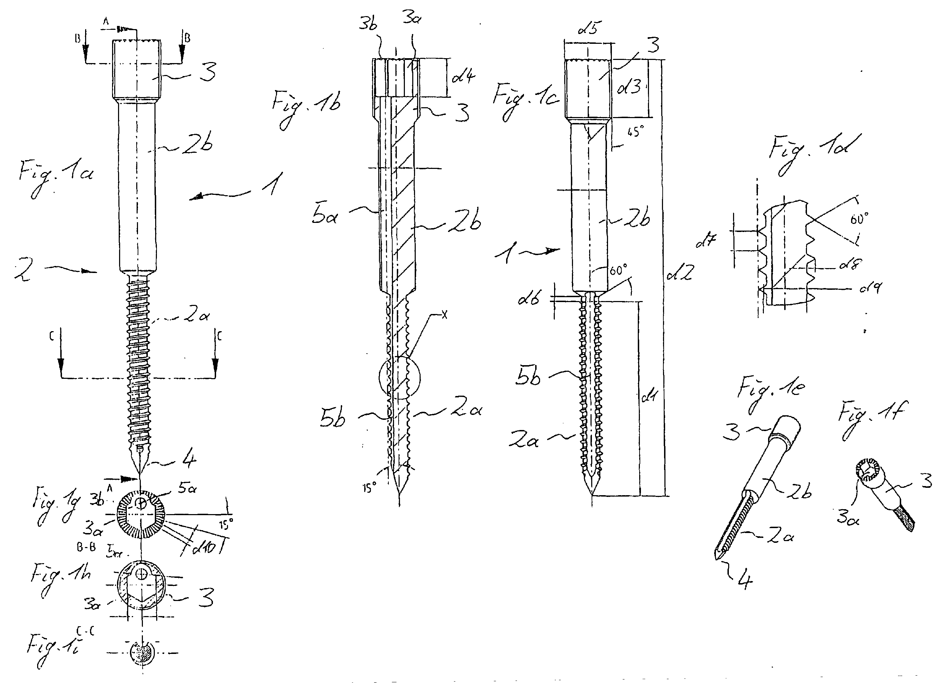

Figur 1 a zeigt in Draufsicht eine Befestigungsvorrichtung 1, welche sich aus einem Schaft 2 und einem Kopf 3 zusammensetzt. Der Schaft 2 weist einen als Schraube ausgebildeten Eingriffbereich 2a und einen Zwischenbereich 2b auf, welcher einen größeren Außendurchmesser als der Eingriffbereich 2a hat. Die Übergangsbereiche zwischen Eingriffbereich 2a, Zwischenbereich 2b und Kopf 3 sind konisch als Kegelabschnitt ausgebildet, so dass die jeweiligen Bereiche ohne scharfe Kanten ineinander übergehen. Am unteren Ende des Eingriffbereiches 2a ist eine Spitze 4 ausgebildet, welche mehrere seitliche Kanten aufweist, wie aus Figur 1e erkennbar. Die Spitze 4 vereinfacht das Ansetzen und Einbringen der Befestigungsvorrichtung 1.

Figur 1b zeigt einen Querschnitt der in Figur 1 gezeigten Befestigungsvorrichtung entlang der Linie A-A. Dabei ist die als Führung für ein Sicherungselement dienende Bohrung 5a in dem Zwischenbereich 2b und dem Kopf 3 erkennbar, wobei die Führung, wie durch die strichpunktierte Linie angedeutet, außerhalb des Durchgangsloches 5a im Bereich des Eingriffbereiches 2a als Rille oder Ausnehmung 5b ausgebildet ist, so dass ein Sicherungselement durch den Kopf 3 und den Zwischenbereich 2b hindurch entlang des Eingriffbereiches 2a gerührt werden kann und so zum Beispiel nach Eindringen der Befestigungsvorrichtung zum Beispiel in einen Knochen in etwa parallel zur Eindringrichtung der Befestigungsvorrichtung durch die Führung 5a, 5b geführt ebenfalls in den Knochen eingebracht werden kann, um so eine Verdrehsicherung der Befestigungsvorrichtung zu erhalten.

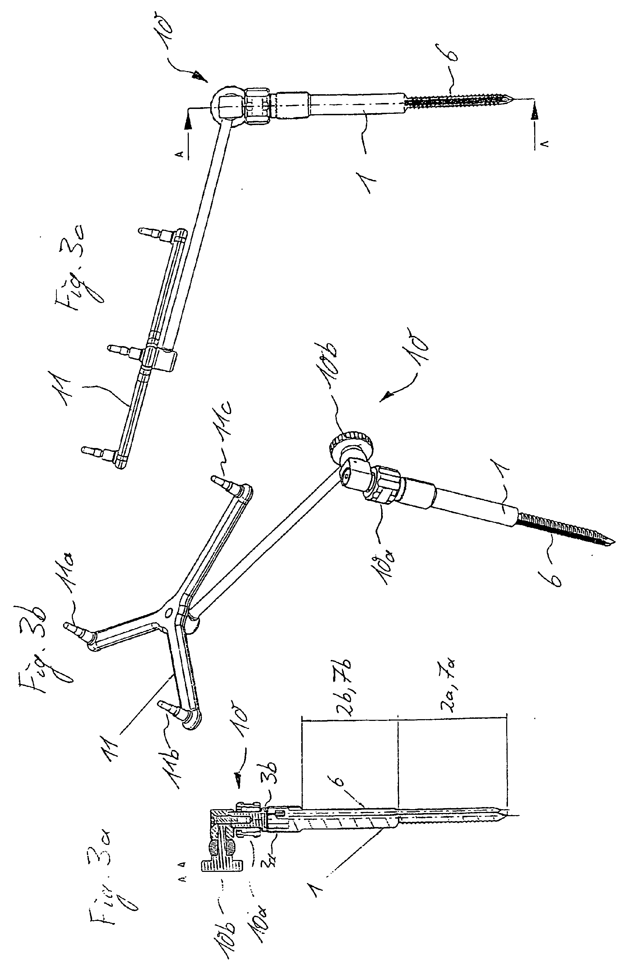

Der Kopf 3 weist eine auch in der perspektivischen Ansicht von Figur 1f und der Draufsicht von Figur 1g erkennbare Vertiefung 3a auf, welche im Querschnitt in etwa ein gleichförmiges Sechseck bildet, um zum Beispiel einen Inbusschlüssel oder ein Aufsteckelement ansetzen zu können. An einer Seite ist die Vertiefung 3a etwa halbrundförmig ausgebildet, um das Einbringen eines Sicherungselements in das Führungsloch 5a zu ermöglichen, wie in Figur 3a gezeigt.

Figur 1c zeigt die in Figur 1a gezeigte und um 180 ° gedrehte Befestigungsvorrichtung 1. Dabei ist die im Eingriffbereich 2a verlaufende Rille oder Führungsausnehmung 5b erkennbar, welche den Gewindegang der Schraube unterbricht, so dass ein Sicherungselement parallel zur Schraube geführt werden kann.

Beispielhafte Maße für die erfindungsgemäße Befestigungsvorrichtung können gemäß einer bevorzugten Ausführungsform der Befestigungsvorrichtung wie folgt sein: Länge des Eingriffbereichs 2a d1 = 40 mm, Gesamtlänge der Befestigungsvorrichtung 1 d2 = 90 mm; Länge des Kopfes 3 d3 = 12 mm; Tiefe der Ausnehmung 3a im Kopf 3 d4 = 8 mm und Durchmesser des Kopfes 3 d5 = 10 mm. Eine Kante der Spitze 4 ist zum Beispiel um 15 ° geneigt gegen die Längsachse der Befestigungsvorrichtung ausgebildet. Der Übergangsbereich zwischen Eingriffbereich 2a und Zwischenbereich 2b weist einen kleinen Abstandsbereich der Länge d6 = 1 mm auf, an welchem sich ein kleines kegelförmiges Anschlussstück mit einem Winkel der Mantelfläche des Kegelstumpfes von 60° bzgl. der Längsachse anschließt. Figur 1d zeigt den in Figur 1b gekennzeichneten Ausschnitt X des mit einem Gewinde versehenen Eingriffbereiches 2a. Dabei ist in der gezeigten Ausführungsform die Ganghöhe des Gewindes d7 = 1,6 mm, wobei das Gewinde um d8 = 0,7 mm herausragt. Der Abstand eines Gewindeganges zu einem in der Schnittebene liegenden Innenbereiches der Schraube d9 beträgt 3,6 mm.

Figur 1g zeigt eine Draufsicht auf die Befestigungsvorrichtung 1, wobei eine Vielzahl von umlaufenden Vertiefungen 3b des Kopfes ersichtlich ist. In der gezeigten Ausführungsform sind innerhalb eines Abstandes d10 = 1 mm zwei kleine Vertiefungen oder Rillen 3b umlaufend um den oberen Bereich des Kopfes 3 angeordnet. Diese umlaufenden Vertiefungen 3b ermöglichen ein verdrehsicheres Anbringen zum Beispiel eines Referenzsterns in einer Vielzahl von Drehpositionen, wobei der Referenzstern in unterschiedlichen Winkelpositionen relativ zum Kopf 3 durch die Vertiefungen 3b verdrehgesichert an der Befestigungsvorrichtung 1 angebracht bzw. arretiert werden kann.

Figur 1h zeigt eine Schnittansicht entlang der Linie B-B der in Figur 1a gezeigten Befestigungsvorrichtung. Dabei ist wie auch in Figur 1g ersichtlich der Außenumfang der Vertiefung 3a so ausgelegt, dass einerseits durch das als Führung dienende Durchgangsloch 5a ein Sicherungselement eingebracht werden kann und anschließend nach Einbringen des Sicherungselements durch die mehrere Kanten aufweisende Vertiefung 3a ein Aufsatz auf der Befestigungsvorrichtung 1 verdrehsicher aufgesetzt werden kann.

Figur 1i zeigt einen Querschnitt entlang der in Figur 1 gezeigten Linie C-C des Eingriffbereiches 2a.

Die oben angegebenen konkreten Abmessungen der in Figur 1 gezeigten Ausführungsform einer erfindungsgemäßen Befestigungsvorrichtung 1 sind nur als Beispiele zur Erläuterung der Erfindung anzusehen und sollen verdeutlichen, dass die erfindungsgemäße Befestigungsvorrichtung zum Beispiel das ortsfeste anbringen eines Referenzsterns an einem Knochen mittels eines äußerst geringen chirurgischen Eingriffes ermöglicht.

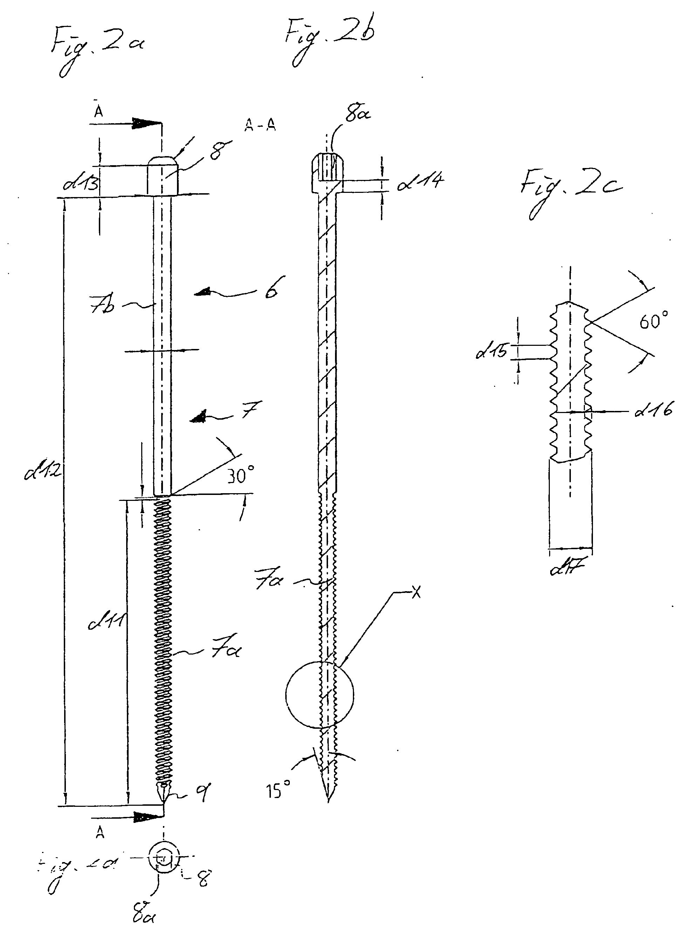

Die Figuren 2a bis 2d zeigen ein Ausführungsbeispiel eines Sicherungselements 6, welches zusammen mit der erfindungsgemäßen in Figur 1 gezeigten Befestigungsvorrichtung 1 verwendet werden kann. Das Sicherungselement 6 weist einen Schaft 7 und einen Kopf 8 auf. Der Schaft 7 unterteilt sich in einen mit einem Schraubengewinde versehenen Eingriffbereich 7a, an dessen unterem Ende eine Spitze 9 vorgesehen ist und der an dem der Spitze 9 gegenüberliegenden Ende an den Zwischenbereich 7b angrenzt, welcher zwischen dem Eingriffbereich 7a und dem Kopf 8 liegt. Der Kopf 8 weist wiederum eine Vertiefung 8a auf, welche wie in der Draufsicht von Figur 2d gezeigt in Form eines Sechsecks ausgebildet ist, um zum Beispiel einen Inbusschlüssel an dem Kopf 8 des Sicherungselements 6 anzusetzen, um das Sicherungselement 6 durch die Führung 5 der zum Beispiel in einen Knochen eingeschraubten Befestigungsvorrichtung 1 einzuschrauben und so wie zum Beispiel in der Querschnittsansicht von Figur 3a gezeigt ein Verdrehen der Befestigungsvorrichtung 1 zu verhindern. Bei der gezeigten Ausführungsform wird im allgemeinen eine Verdrehsicherung dadurch erreicht, dass nach Einbringen des Sicherungselements 6 in die Befestigungsvorrichtung 1 die nebeneinander liegenden Eingriffbereiche 2a und 7a ein nicht mehr rotationssymmetrisches Gebilde darstellen, so dass eine Drehung der Befestigungsvorrichtung 1 durch das Sicherungselement 6 verhindert wird. Ein eventuelles Drehen des Sicherungselements 6 wirkt sich jedoch nicht auf die Position der Befestigungsvorrichtung 1 aus.

Beispielhafte Abmessungen des in Figur 2 gezeigten Sicherungselements 6 sind: Länge des Eingriffbereiches 7a d11= 40 mm; Länge des Schaftes 7 d12 = 80 mm; Länge des Kopfes 8 d13 = 4 mm und Länge des Kopfes 8, in dem keine Vertiefung 8a vorgesehen ist d14 = 1,5 mm.

Die angegebenen Bemaßungen sind nur als beispielhafte Ausführungsformen anzusehen und sollen verdeutlichen, dass mit erfindungsgemäßen Vorrichtung nur ein relativ kleiner chirurgischer Eingriff erforderlich ist. Es ist möglich, dass die Befestigungsvorrichtung 1 und das Sicherungselement 6 in etwa die gleiche Länge aufweisen und bezüglich der Eingriffbereiche 2a, 7a und Zwischenbereiche 2b und 7b in etwa ähnliche Abmessungen aufweisen. Jedoch kann auch ein längeres oder kürzeres Sicherungselement mit einem längeren oder kürzeren Eingriffbereich 7a und/oder Zwischenbereich 7b verwendet werden. Allgemein ist es vorteilhaft, wenn das Sicherungselement so ausgebildet ist, dass die Gesamtlänge einschließlich des Kopfes 8 kleiner ist als die Gesamtlänge der Befestigungsvorrichtung 1, so dass wenn das Sicherungselement 6 nicht mehr aus dem Kopf 3 herausragt, insbesondere vollständig in die Befestigungsvorrichtung 1 eingebracht ist, d.h. die Spitzen 4 und 9 in etwa nebeneinander liegen, der Kopf 8 des Sicherungselements 6 im unteren Bereich des Kopfes 3 der Befestigungsvorrichtung 1 versenkt werden kann, so dass die vollständige Vertiefung 3a des Kopfes 3 zum Einbringen zum Beispiel eines Referenzsterns verwendet werden kann, ohne dass der Kopf 8 des eingebrachten Sicherungselements 6 in die Vertiefung 3a hineinragt.

Figur 2c zeigt eine Vergrößerung des in Figur 2b mit X gekennzeichneten Eingriffbereichs 7a. Die Ganghöhe d15 des Gewindes ist in der gezeigten Ausführungsform 0,75 mm; das Gewinde steht um d16 = 0,4 mm von der Außenoberfläche des Eingriffbereichs 7a vor und der Au-ßendurchmesser des Gewindes beträgt d17 = 2,3 mm.

Die Figuren 3a bis 3d zeigen ein erfindungsgemäßes Positionierungssystem, wobei das in Figur 2 gezeigte Sicherungselement 6 in die in Figur 1 gezeigte Befestigungsvorrichtung eingesetzt ist.

Figur 3c zeigt eine Draufsicht auf ein Positionierungssystem, wobei auf dem Befestigungssystem 1, 6 ein als Positionierungselement dienender Referenzstern 11 aufgesetzt ist, auf dessen Haltevorrichtungen 11a, 11b und 11c jeweils Marker aufgesteckt werden können. Der Referenzstern 11 ist mittels einer verstellbaren Ausrichtungsvorrichtung 10 an dem Befestigungssystem 1, 6 befestigt.

Figur 3b zeigt dass in Figur 3c gezeigte Positionierungssystem in perspektivischer Ansicht. Die Ausrichtungsvorrichtung 10 weist eine Schraube 10a auf, welche zur Befestigung an dem Befestigungssystem 1, 6 und/oder zur Drehung des daran befestigten Referenzsterns 11 um die Längsachse der Befestigungsvorrichtung 1 verwendet werden kann. Über eine weitere Schraube 10b kann der Referenzstern 11 um eine Achse gedreht werden, welche in etwa senkrecht zur Mittelachse der Befestigungsvorrichtung 1 ist. Hierdurch kann unter Verwendung der Ausrichtungsvorrichtung 10 der Referenzstern 11 an einem zum Beispiel in einen Knochen eingebrachtem Befestigungssystem 1, 6 angebracht und für eine gute Erfassung der auf den Referenzstern 11 aufgebrachten Markern (nicht gezeigt) positioniert werden.

Figur 3a zeigt ein Schnittbild entlang der Linie A-A des in Figur 3c gezeigten Positionierungssystems. Dabei ist ersichtlich, dass im eingesetzten Zustand des Sicherungselements 6 in der Befestigungsvorrichtung 1 die Eingriffbereiche 2a, 7a und die Zwischenbereiche 2b, 7b in etwa nebeneinander liegen. Die Ausrichtungsvorrichtung 10 ist in die Vertiefung 3a der Befestigungsvorrichtung eingesetzt und daran befestigt. Durch Vorsprünge und/oder Vertiefungen auf der Unterseite der Ausrichtungsvorrichtung 10, welche in die Vorsprünge bzw. Vertiefungen 3b auf der Oberseite der Befestigungsvorrichtung 1 eingreifen, kann eine Verdrehsicherung zwischen Ausrichtungsvorrichtung 10 mit daran befestigtem Referenzstern 11 und dem Befestigungssystem 1, 6 erreicht werden.

Die vorliegende Erfindung ermöglicht somit das relativ einfache Einbringen einer verdrehgesicherten Befestigungsvorrichtung zum Beispiel in einen Knochen unter Verwendung von zum Beispiel nur zwei Teilen, der Befestigungsvorrichtung und mindestens einem Sicherungselement. Hierdurch verringern sich die Herstellungskosten für ein solches Befestigungssystem. Weiterhin ist die Reinigung eines solchen Befestigungssystems aufgrund der Verwendung von z.B. nur zwei Teilen relativ einfach.

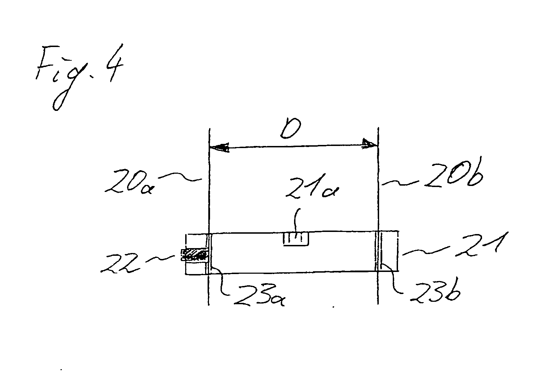

Figur 4 zeigt ein alternatives Befestigungssystem.

Zur Befestigung eines Positionierungselements, wie zum Beispiel des in Figur 3 gezeigten Referenzsternes 11 an einem Element, wie zum Beispiel einem Knochen, können auch an sich bekannte Kirschnerdrähte oder Schanzschrauben 20a, 20b verwendet werden, welche auf bekannte Weise in einen Knochen eingebracht werden. Der Abstand D dazwischen kann zum Beispiel 0,1 bis 100 mm, bevorzugt 0,1 bis 10 mm betragen. Entlang der Kirschnerdrähte oder Schanzschrauben 20a, 20b wird eine Befestigungsvorrichtung 11 geführt oder aufgesteckt, welche durchgehende Bohrungen 23a, 23b für die Drähte oder Schrauben 20a, 20b aufweist. Mittels eines oder mehrer Arretierelemente 22, die zum Beispiel verschoben werden können, um eine kraftschlüssige Verbindung mit einem oder mehreren Kirschnerdrähten oder Schanzschrauben herzustellen, kann die Befestigungsvorrichtung 21 verdrehsicher an den Drähten oder Schrauben 20a, 20b befestigt werden. Ist die Befestigungsvorrichtung 21 durch die Betätigung eines Arretierelements 22 positioniert, so können überstehende Drähte oder Schrauben abgeschnitten werden und an die Befestigungsvorrichtung 21 kann ein Positionierungselement angebracht werden, in eine Vertiefung 21a eingesteckt und zum Beispiel mittels geeigneter Schrauben befestigt werden.

Durch zwei im Abstand D voneinander in eine Knochen eingebrachte Elemente, wie zum Beispiel Kirschnerdrähte oder Schanzschrauben, kann die Befestigungsvorrichtung 21 ortsfest und verdrehsicher an einem Element, wie zum Beispiel einem Knochen, angebracht werden.

## Patentansprüche

1. Befestigungsvorrichtung, die
a) einen Eingriffbereich (2a) umfasst, der in ein Objekt eingebracht werden kann und mindestens eine Führung (5a, 5b) für mindestens ein Sicherungselement (6) aufweist,
b) wobei bei einem eingebrachten Eingriffbereich (2a) das Sicherungselement (6) an der Führung (5a, 5b) des eingebrachten Eingriffbereichs (2a) entlanggeführt werden kann und
c) wobei bei einem eingebrachten Sicherungselement (6) die Befestigungsvorrichtung gegen Verdrehen gesichert ist,
**dadurch gekennzeichnet, dass**
d) die Führung als Bohrung (5a) und/oder als Ausnehmung (5b) ausgebildet ist.

2. Befestigungsvorrichtung nach Anspruch 1, wobei die Befestigungsvorrichtung einen Schaft (2) und einen Kopf (3) aufweist.

3. Befestigungsvorrichtung nach Anspruch 2, wobei der Schaft (2) den Eingriffbereich (2a) und einen Zwischenbereich (2b) aufweist.

4. Befestigungsvorrichtung nach Anspruch 3, wobei der Durchmesser des Zwischenbereiches (2b) größer als der Durchmesser des Eingriffbereiches (2a) ist.

5. Vorrichtung nach einem der vorhergehenden Ansprüche, wobei zwei, drei oder mehr Führungen (5) in der Befestigungsvorrichtung ausgebildet sind.

6. Befestigungsvorrichtung nach einem der vorhergehenden Ansprüche, wobei die Befestigungsvorrichtung einen mit einem Gewinde versehenen Bereich aufweist.

7. Befestigungsvorrichtung nach einem der vorhergehenden Ansprüche, wobei die Befestigungsvorrichtung einen als Nagel ausgebildeten Bereich und/oder einen Bereich mit in etwa parallel zur Einbringrichtung verlaufenden Kanten aufweist.

8. Befestigungsvorrichtung nach einem der vorhergehenden Ansprüche, wobei die Befestigungsvorrichtung ein spitz zulaufendes Element (4) am unteren Ende aufweist.

9. Befestigungsvorrichtung nach einem der vorhergehenden Ansprüche, wobei die Befestigungsvorrichtung ein Verbindungselement oder eine Vertiefung (3a) aufweist, bei welcher Werkzeuge angesetzt oder Vorrichtungen (10, 11) angebracht werden können.

10. Befestigungssystem mit einer Befestigungsvorrichtung (1) nach einem der Ansprüche 1 bis 9 und mindestens einem Sicherungselement (6), welches zumindest teilweise in die Führung (5a,5b) eingesetzt ist.

11. Befestigungssystem nach Anspruch 10, wobei das Sicherungselement (6) einen Bereich (7a) aufweist, welcher mit einem Gewinde versehen ist und/oder einen Bereich (7b) aufweist, welcher eine im wesentlichen glatte Oberfläche aufweist und/oder einen Kopf (8) aufweist.

12. Befestigungssystem nach Anspruch 10 oder 11, wobei die Führung (5a, 5d) der Befestigungsvorrichtung (1) und das Sicherungselement (6) so ausgebildet sind, dass das in die Befestigungsvorrichtung (1) eingesetzte Sicherungselement (6) im Wesentlichen kein Spiel aufweist.

13. Positionierungssystem mit einem Befestigungssystem (1, 6) nach einem der Ansprüche 10 bis 12 und einem daran befestigten Positionierungselement (11).

14. Positionierungssystem nach Anspruch 13, wobei das Positionierungselement (11) mittels einer verstellbaren Ausrichtungsvorrichtung (10) an dem Befestigungssystem (1, 6) befestigt ist.

15. Positionierungssystem nach einem der Ansprüche 13 oder 14, wobei das Positionierungselement ein Referenzstern (11) ist.

## Claims

1. A fixing device
a) including an operative section (2a) which can be inserted into an object and comprises at least one guide (5a, 5b) for at least one securing element (6),
b) wherein when an operative section (2a) is inserted, the securing element (6) can be guided along the guide (5a, 5b) of the inserted operative section (2a), and
c) wherein when a securing element (6) is inserted, the fixing device is secure against turning,
**characterised in that**
d) the guide is formed as a bore (5a) and/or as a recess (5b).

2. The fixing device as set forth in claim 1, wherein the fixing device comprises a shaft (2) and a head (3).

3. The fixing device as set forth in claim 2, wherein the shaft (2) comprises the operative section (2a) and an intermediate section (2b).

4. The fixing device as set forth in claim 3, wherein the diameter of the intermediate section (2b) is larger than the diameter of the operative section (2a).

5. The device as set forth in any one of the preceding claims, wherein two, three or more guides (5) are formed in the fixing device.

6. The fixing device as set forth in any one of the preceding claims, wherein the fixing device comprises a section provided with a thread.

7. The fixing device as set forth in any one of the preceding claims, wherein the fixing device comprises a section formed as a nail and/or a section with edges running approximately parallel to the direction of insertion.

8. The fixing device as set forth in any one of the preceding claims, wherein the fixing device comprises a tapered element (4) at its lower end.

9. The fixing device as set forth in any one of the preceding claims, wherein the fixing device comprises a connecting element or a recess (3a), wherein tools can be positioned or devices (10, 11) can be attached.

10. A fixing system comprising a fixing device (1) as set forth in any one of claims 1 to 9 and at least one securing element (6) which is at least partially inserted into the guide (5a, 5b).

11. The fixing system as set forth in claim 10, wherein the securing element (6) comprises a section (7a) provided with a thread and/or comprises a section (7b) comprising a substantially smooth surface and/or comprises a head (8).

12. The fixing system as set forth in claim 10 or 11, wherein the guide (5a, 5d) of the fixing device (1) and the securing element (6) are formed in such a way that the securing element (6) inserted into the fixing device (1) exhibits substantially no play.

13. A positioning system comprising a fixing system (1, 6) as set forth in any one of claims 10 to 12 and a positioning element (11) fixed to it.

14. The positioning system as set forth in claim 13, wherein the positioning element (11) is fixed to the fixing system (1, 6) by means of an adjustable aligning device (10).

15. The positioning system as set forth in any one of claims 13 or 14, wherein the positioning element is a reference star (11).

## Revendications

1. Dispositif de fixation qui
a) comprend une zone de prise (2a) qui peut être introduite dans un objet et qui présente au moins un guide (5a, 5b) pour au moins un élément de sûreté (6),
b) l'élément de sûreté (6), lorsque une roue de prise (2a) est introduite, pouvant être guidé le long du guide (5a, 5b) de la zone de prise (2a) introduite et
c) le dispositif de fixation étant bloqué contre une rotation lorsque l'élément de sûreté (6) est introduit,
**caractérisé en ce que**
d) le guide est réalisé sous la forme d'un perçage (5a) et/ou d'un évidement (5b).

2. Dispositif de fixation selon la revendication 1, dans lequel le dispositif de fixation comporte une tige (2) et une tête (3).

3. Dispositif de fixation selon la revendication 2, dans lequel la tige (2) présente la zone de prise (2a) et une zone intermédiaire (2b).

4. Dispositif de fixation selon la revendication 3, dans lequel le diamètre de la zone intermédiaire (2b) est supérieur au diamètre de la zone de prise (2a).

5. Dispositif de fixation selon l'une des revendications précédentes, dans lequel deux, trois guides (5) ou plus sont réalisés dans le dispositif de fixation.

6. Dispositif de fixation selon l'une des revendications précédentes, dans lequel le dispositif de fixation présente une zone pourvue d'un filetage.

7. Dispositif de fixation selon l'une des revendications précédentes, dans lequel le dispositif de fixation présente une zone réalisée sous la forme d'un clou et/ou une zone avec des bords s'étendant approximativement parallèlement à la direction d'introduction.

8. Dispositif de fixation selon l'une des revendications précédentes, dans lequel le dispositif de fixation présente un élément (4) en forme de pointue à l'extrémité inférieure.

9. Dispositif de fixation selon l'une des revendications précédentes, dans lequel le dispositif de fixation comporte un élément de liaison ou un renfoncement (3a) dans lequel des outils peuvent être placés ou des dispositifs (10, 11) fixés.

10. Système de fixation comportant un dispositif de fixation (1) selon l'une des revendications 1 à 9 et au moins un élément de sûreté (6) qui est inséré au moins en partie dans le guide (5a, 5b).

11. Système de fixation selon la revendication 10, dans lequel l'élément de sûreté (6) présente une zone (7a) qui est pourvue d'un filetage et/ou une zone (7b) qui présente une surface sensiblement lisse et/ou une tête (8).

12. Système de fixation selon la revendication 10 ou 11, dans lequel le guide (5a, 5d) du dispositif de fixation (1) et l'élément de sûreté (6) sont réalisés de manière que l'élément de sûreté (6), introduit dans le dispositif de fixation (1), ne présente sensiblement pas de jeu.

13. Système de positionnement comportant un système de fixation (1, 6) selon l'une des revendications 10 à 12 et un élément de positionnement (11) fixé sur celui-ci.

14. Système de positionnement selon la revendication 13, dans lequel l'élément de positionnement (11) est fixé sur le système de fixation (1, 6) au moyen d'un dispositif d'orientation (10) réglable.

15. Système de positionnement selon l'une des revendications 13 ou 14, dans lequel l'élément de positionnement est une étoile de référence (11).
